# EUROPEAN PATENT SPECIFICATION

(11) **EP 3 570 860 B1**
(45) Date of publication and mention of the grant of the patent: **13.10.2021**
(21) Application number: 18711183.6
(22) Date of filing: 19.01.2018
(51) Int. Cl.: A61K 36/07, A61P 35/00

(54) **ANTICANCER HETEROBASIDION ANNOSUM EXTRACT, COMPOSITIONS AND USES THEREOF**
KREBSHEMMENDER HETEROBASIDION ANNOSUM EXTRAKT, ZUSAMMENSETZUNGEN UND VERWENDUNGEN DAVON
EXTRAIT ANTICANCEREUX D'HETEROBASIDION ANNOSUM, COMPOSITIONS ET UTILISATIONS DE CEUX-CI

(30) Priority: 20.01.2017 PL 42026717
(43) Date of publication of application: 27.11.2019
(73) Proprietor: Uniwersytet Medyczny w Bialymstoku, 15-089 Bialystok (PL); Politechnika Bialostocka, 15-351 Bialystok (PL)
(72) Inventor: CAR, Halina, 15-562 Bialystok (PL); SADOWSKA, Anna, 15-642 Bialystok (PL); MACIEJCZYK, Mateusz, 26-600 Radom (PL); BAKIER, Slawomir, 15-523 Grabówka (PL); ZAPORA, Ewa, 15-558 Bialystok (PL); WOLKOWYCKI, Marek, 17-200 Hajnówka (PL); SURAZYNSKI, Arkadiusz, 15-687 Bialystok (PL)
(74) Representative: Kawczynska, Marta Joanna
(86) International application number: PCT/PL2018/000007
(87) International publication number: WO 2018/135958

(56) References cited:
- WO-A1-2016/161138
- US-A1- 2006 045 887

## Description

The invention is defined in claims 1-6 and provides an extract from a polypore fungus, a composition comprising this extract and the use thereof in the fields of medicine and pharmacy.

Colorectal cancer has become one of the most common gastrointestinal malignancies in Poland. The incidence of this cancer has increased dramatically since the early 1990s. Surgical treatment followed by radiation therapy and/or chemotherapy is currently administered to patients with colorectal cancer. However, the commercially available synthetic anticancer drugs used during chemotherapy are not specifically targeted. They show significant toxicity and multiple adverse reactions, because their cytostatic activity affects not only cancer cells, but also normal cells. In addition, their high prices contribute to the high cost of therapy. Therefore, there is a strong demand for effective and less toxic anticancer agents composed of natural ingredients which would involve lower financial burden due to the availability of the active substance.

A number of studies conducted mainly in Japan, China and the USA reveal high therapeutic activity of extracts and compounds obtained from polypore fungi. They exhibit varied pharmacological activity confirmed in experimental research.

In view of the above, the purpose of the invention was to develop an extract from a polypore fungus which could be used as an anticancer medicine of natural origin, having effective activity against cancer cells and low cytotoxicity towards normal cells and which would contribute to lower use of synthetic chemotherapeutic agents, thus reducing the cost of anticancer therapy and side effects thereof.

An extract from *Heterobasidion annosum* (HA) is obtained using a process comprising the following stages:
a) preparing a raw material by pulverising a fresh sporocarp of *Heterobasidion annosum* fungus to a particle size lower than 2 mm so that its temperature does not increase above 40°C;
b) extracting *Heterobasidion annosum* by pouring methanol in a ratio of 1:2 (raw material : solvent, mass : volume (m:V)) on the raw material, stirring the obtained mixture for a period of from 24 to 120 hours, decanting the methanol and adding a fresh portion of methanol in a ratio of 1:2 (raw material : solvent, m:V);
c) repeating the said extraction procedure at least 3 times;
d) combining and filtrating the obtained extracts through filter paper; and
e) evaporating the solvent to obtain an extract in the form of powder.

The *Heterobasidion annosum* extract is used as a medicine, in particular as an anticancer medicine. The extract is preferably used for the prevention and/or treatment of colorectal cancer. According to the invention, the *Heterobasidion annosum* extract is used at dose from 1 to 10 mg/kg body weight.

The *Heterobasidion annosum* extract is also used for the manufacture of a medicine for the prevention and/or treatment of colorectal cancer.

The invention also provides a pharmaceutical composition comprising an active ingredient and an auxiliary agent in which the active ingredient is the *Heterobasidion annosum* extract and the auxiliary agent is a carrier. Extract concentration in the composition of the invention is from 0.1 to 5 µg/mL.

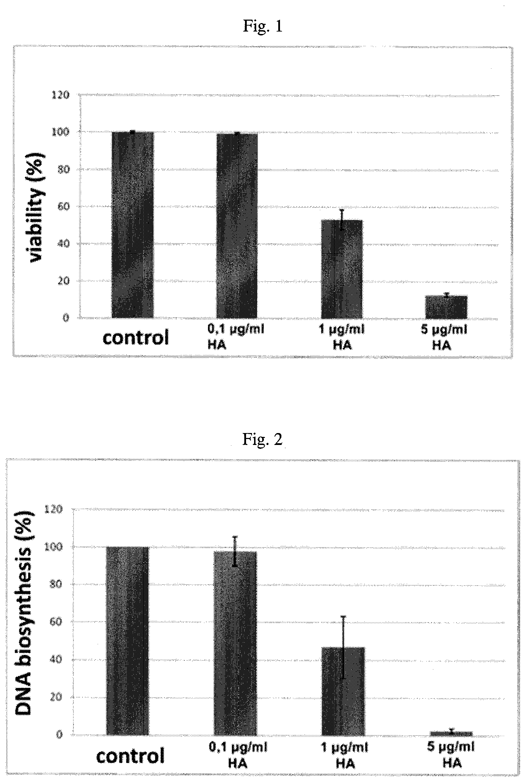

*Heterobasidion annosum* is a fungus species widespread in the whole temperate zone and, thus, it is readily available and the process for the manufacture of the extract thereof is not expensive. In addition, the *Heterobasidion annosum* extract has strong anticancer properties and at the same time exhibits weak cytostatic activity towards normal cells, as illustrated in Figures 1-7.
Figure 1 illustrates the effect of the *Heterobasidion annosum* fungus extract on the viability of the DLD-1 cancer cell line.
Figure 2 illustrates the effect of the *Heterobasidion annosum* fungus extract on DNA biosynthesis in the DLD-1 cancer cell line.
Figure 3 illustrates the effect of the *Heterobasidion annosum* fungus extract on the viability of CRL-1474 fibroblast cell line.
Figure 4 illustrates the effect of the *Heterobasidion annosum* fungus extract on DNA biosynthesis of CRL-1474 fibroblast cell line.
Figure 5 shows the results of mitochondrial potential tests in the DLD-1 cell line culture.
Figure 6 shows the results of apoptosis tests in the DLD-1 colorectal cancer cell line.
Figure 7 shows the mean tumour volume over time in the control group (without treatment) and in the groups administered with the *Heterobasidion annosum* extract or 5-fluorouracil (5FU).

The invention is illustrated with the following examples.

### Example I. Manufacture of the extract from Heterobasidion annosum

A fresh sporocarp (fungi) harvested in the Bialowieza Forest from the trunks of infested pine trees was pulverised to a particle size lower than 2 mm. Its temperature was controlled during pulverisation, so that it did not increase above 40°C. 200 mL of methanol was poured over 100 g of the raw material (sporocarp to solvent ratio: 1:2; m:V). This was placed on a magnetic stirrer. After stirring for 24 hours, the solvent was decanted and a portion of 200 mL of fresh methanol was added. The extraction was repeated 3 times. The methanol extracts were combined and filtered through filter paper. The solvent was evaporated using a vacuum evaporator. The resulting dry extract was in the form of cream-coloured powder.

### Example II. Manufacture of a composition comprising the extract from Heterobasidion annosum

Appropriate quantities of the dry extract (powder) prepared as above were weighed out and combined with dimethylsulfoxide (DMSO) or phosphate buffered normal saline (PBS), and compositions were prepared in which the concentrations of the extract were 0.1 µg/mL, 1 µg/mL and 5 µg/mL.

Other carriers used in compositions comprising the *Heterobasidion annosum* extract are normal saline or isotonic glucose solution.

### Example III. Biological tests

### a) Tests on cell cultures

The *Heterobasidion annosum* extract in the form of a DMSO solution was used in the tests on cell cultures. Extract concentration was 0.1; 1 and 5 µg/mL.

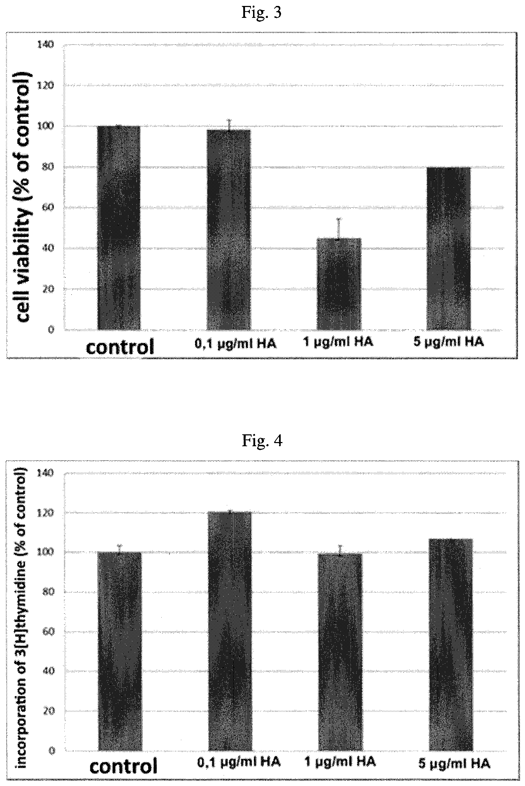

Cytotoxic properties of the extract were tested in the DLD-1 colorectal cancer cell line. The tests were performed using three concentrations (0,1; 1 and 5 µg/mL), selected as a result of multiple assays based on different concentrations. Cytotoxicity was evaluated using the tetrazolium salt (MTT) reduction assay, and DNA biosynthesis was evaluated using a radioisotope method with labelled thymidine. The assays were performed after 24-hour cell incubation with the extract from the said fungus. The results showed a significant decrease in the viability of cancer cells with increasing doses of the applied extract (Figure 1). The results which illustrate the incorporation of labelled thymidine (DNA biosynthesis) into cancer cells (Figure 2) are compatible with those obtained in the MTT assay and they confirm that the extract is active in the range tested. The results obtained in the test of CRL-1474 fibroblast cell line (two replicates of independent tests) indicate a minimum decrease in fibroblast viability after 24-hour incubation with the use of extract at concentration of 0.1 µg/mL, and over 50% decrease in viability with the use of extract at concentration of 1 µg/mL; however, when the extract at concentration of 5 µg/mL was used, cell viability decreased by only 20% compared to the control (Figure 3). These results partially correspond to the data obtained in the DNA biosynthesis assay which indicated sustained replication in approx. 100% of cells (Figure 4).

It can be concluded based on the low cytotoxicity towards very common physiological cells (fibroblasts) that adverse effects related to administration of the extract should be limited.

In addition, tests of the mitochondrial potential and apoptosis in the DLD-1 cell line culture were performed. The test was performed using the NucleoCounter® NC-3000™ fluorescence microscope.

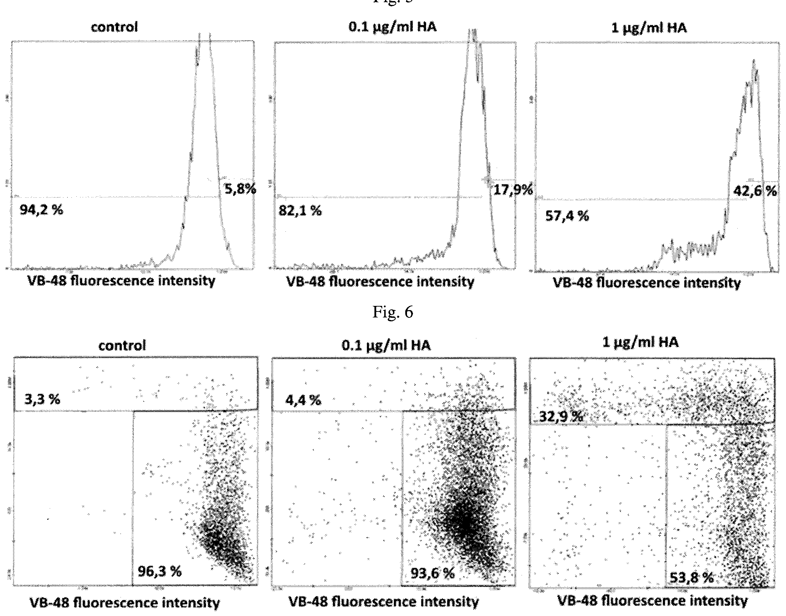

The mitochondrial potential was evaluated using the VB-48 dye. The population of living cells can be determined by measuring fluorescence intensity of the dye. As can be observed in the chart shown in Figure 5 in which the horizontal axis is fluorescence intensity of VB-48, and the vertical axis is % of cells, the highest percentage of cells with normal mitochondrial polarisation is present in the control group (94.2%). 24 hours after adding the *Heterobasidion annosum* (HA) fungus extract to the cells, an increased percentage of cells with depolarised mitochondrial membranes was observed with increasing concentration (17.9% at 0.1 µg/mL HA concentration and 42.6% at 1 µg/mL HA concentration).

The above results confirm the results obtained from the apoptosis tests in the DLD-1 colorectal cancer cell line. The results of the apoptosis tests are illustrated in Figure 6. The tests involved measurements of the fluorescence intensity of propidium iodide (PI) which enters dead cells (vertical axis) and fluorescence intensity of the VB-48 dye (horizontal axis). As shown in Figure 6, 96.3% of cells that did not undergo apoptosis and approx. 3.3% of apoptotic cells were found in the control group. After the HA extract was added at concentrations of 0.1 and 1 µg/mL, the percentage of cells undergoing apoptosis increased after 24 hours: 93.6% and 53.8% of cells that did not undergo apoptosis and 4.4% and 32.9% of apoptotic cells, respectively.

In addition, changes in protein expression were visualised and intracellular protein translocation in the DLD-1 colorectal cancer cell line was evaluated via tests using a BD Pathway 855 high-throughput fluorescence microscope. Caspase-3 and protein p53 were evaluated. Increasing expression of caspase-3 was observed with increasing extract concentration (0.1; 1 and 5 µg/mL) and its translocation from the cytoplasm to the cell nucleus. In the case of protein p53, its translocation to the cell nucleus was observed, indicating that apoptosis was activated.

### b) Animal studies.

An *in vivo* test on CBy.Cg-Fox1/cmdb strain mice with implanted colorectal cancer cells was performed. Between day 7 after the cancer cells were implanted and day 35 of the experiment, the mice were given the extract in the form of a PBS solution at dose of 1 mg/kg body weight (intragastric administration). The extract can also be administered in the form tablets or capsules or intravenously.

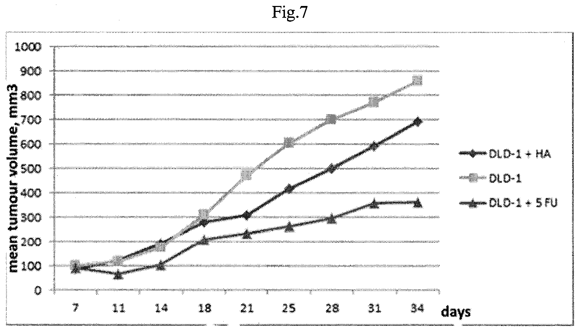

As can be seen in Figure 7, a lower tendency for tumour growth can be observed in the group of mice receiving the *Heterobasidion annosum* (HA) fungus extract compared to the control group which received the carrier only. The lowest tumour growth was noted in the group of animals which received intraperitoneally 5-fluorouracil (5FU), a conventional chemotherapeutic agent. Importantly, the condition of the mice was very good throughout the experiment. No symptoms were found which would indicate toxic effects of the extract being administered.

## Claims

1. An extract from *Heterobasidion annosum* obtained using a process comprising the following stages:
a) preparing a raw material by pulverising a fresh sporocarp of *Heterobasidion annosum* fungus to a particle size lower than 2 mm and controlling that the temperature does not increase above 40°C during the process;
b) extracting *Heterobasidion annosum* by pouring methanol in a ratio of 1:2 (raw material: solvent, mass: volume (m:V)) on the raw material, stirring the obtained mixture for a period of from 24 to 120 hours, decanting the methanol and adding a fresh portion of methanol in a ratio of 1:2 (raw material : solvent, m:V);
c) repeating the extraction procedure at least 3 times;
d) combining and filtrating the obtained extracts; and
e) evaporating the solvent to obtain an extract in the form of powder;
wherein the extract so obtained has anticancer effect.

2. The *Heterobasidion annosum* extract of claim 1 for use as an anticancer medicine.

3. The *Heterobasidion annosum* extract of claim 1 for the use according to claim 2, **characterized in that** it is used for the prevention and/or treatment of colorectal cancer.

4. The *Heterobasidion annosum* extract of claim 1 for the use according to claim 3, **characterised in that** it is used at dose from 1 to 10 mg/kg body weight.

5. A pharmaceutical composition comprising an active anticancer ingredient and an auxiliary agent, **characterised in that** the active anticancer ingredient is the *Heterobasidion annosum* extract of claim 1 and the auxiliary agent is a carrier.

6. The composition according to claim 5, **characterised in that** it comprises the extract at a concentration from 0.1 to 5 µg/mL.

## Patentansprüche

1. Extrakt aus *Heterobasidion annosum* erhalten mit einem Verfahren, das die folgenden Schritte umfasst:
a) Herstellung des Rohmaterials durch Pulverisieren eines frischen Sporokarps von *Heterobasidion annosum* Pilz auf eine Partikelgröße von weniger als 2 mm und Kontrolle der Temperatur, so dass sie während des Prozesses nicht über 40°C steigt;
b) Extraktion von *Heterobasidion annosum* durch Gießen von Methanol auf das Rohmaterial im Verhältnis 1:2 (Rohmaterial: Lösungsmittel, Masse: Volumen (m:V)), Rühren der erhaltenen Mischung für einen Zeitraum von 24 bis 120 Stunden, Dekantation von Methanol und Zugabe einer frischen Portion von Methanol im Verhältnis 1:2 (Rohmaterial: Lösungsmittel, m:V);
c) Wiederholung des Extraktionsvorgangs mindestens 3 Mal;
d) Vereinigung und Filtration der gewonnenen Extrakte; und
e) Verdampfen des Lösungsmittels, um einen Extrakt in Form von Pulver zu erhalten; wobei der so erhaltene Extrakt eine Anti-krebs Wirkung hat.

2. *Heterobasidion annosum*-Extrakt nach Anspruch 1 zur Verwendung als ein Anti-krebs Medikament.

3. *Heterobasidion annosum*-Extrakt nach Anspruch 1 zur Verwendung nach Anspruch 2, **dadurch gekennzeichnet, dass** es zur Vorbeugung und/oder Behandlung von Darmkrebs verwendet wird.

4. *Heterobasidion annosum*-Extrakt nach Anspruch 1 zur Verwendung nach Anspruch 3, **dadurch gekennzeichnet, dass** es in einer Dosis von 1 bis 10 mg/kg Körpergewicht verwendet wird.

5. Pharmazeutische Zusammensetzung, umfassend einen Anti-krebs Wirkstoff und ein Hilfsmittel, **dadurch gekennzeichnet, dass** der Anti-krebs Wirkstoff der *Heterobasidion annosum* Extrakt nach Anspruch 1 ist und der Hilfsstoff ein Träger ist.

6. Zusammensetzung nach Anspruch 5, **dadurch gekennzeichnet, dass** sie den Extrakt in einer Konzentration von 0,1 bis 5 µg/ml umfasst.

## Revendications

1. Extrait de *Heterobasidion annosum* obtenu par un processus comprenant les étapes suivantes :
a) La production de la matière première par la pulverisation de basidiocarpes frais du champignon *Heterobasidion annosum* en particules de taille inférieure à 2 mm, et le contrôle de la température pour l'empêcher d'augmenter au dessus de 40°C pendant le processus;
b) L'extraction de *Heterobasidion annosum* en versant du méthanol sur la matière première en proportion 1:2 (matière première: diluant, masse: volume (m:V)), l'action de melanger du mélange obtenu pendant 24 à 120 heures, la décantation du méthanol et l'ajout d'une portion de méthanol neuf en proportion 1:2 (matière première: diluant, m:V) ;
c) La reprise de la procédure d'extraction 3 fois minimum ;
d) La combinaison et le filtrage des extraits obtenus ; et
e) L'évaporation du diluant et l'obtention d'un extrait sous forme d'une poudre ; l'extrait ainsi obtenu ayant des propriétés anticancéreuses.

2. Extrait de *Heterobasidion annosum* selon la revendication 1 à utiliser comme un médicament anticancéreux.

3. Extrait de *Heterobasidion annosum* selon la revendication 1 à utiliser selon la revendication 2, **caractérisé en ce qu'**il est utilisé pour la prévention et/ou le traitement du cancer colorectal.

4. Extrait de *Heterobasidion annosum* selon la revendication 1 à utiliser selon la revendication 3, **caractérisé en ce que** sa dose d'utilisation est comprise entre 1 et 10 mg/kg de la masse corporelle.

5. Composition pharmaceutique contenant un composant actif anticancéreux et un adjuvant, **caractérisée en ce que** le composant actif anticancéreux est un extrait de *Heterobasidion annosum* selon la revendication 1, et l'adjuvant est le vecteur.

6. Composition pharmaceutique la revendication 5, **caractérisée en ce qu'**elle contient un extrait à concentration de 0,1 à 5 µg/ml.
